# EUROPEAN PATENT SPECIFICATION

(11) **EP 2 957 301 B1**
(45) Date of publication and mention of the grant of the patent: **05.04.2017**
(21) Application number: 14173318.8
(22) Date of filing: 20.06.2014
(51) Int. Cl.: A61L 2/00, A61L 9/00

(54) **Method for the control of odour**
Verfahren zur Kontrolle von Gerüchen
Procédé pour le contrôle des odeurs

(43) Date of publication of application: 23.12.2015
(73) Proprietor: Omya International AG, 4665 Oftringen (CH)
(72) Inventor: Ohr, Steffen, 6210 Sursee (CH); Hunziker, Philipp, 4461 Böckten (CH); Schoelkopf, Joachim, 5727 Oberkulm (CH); Gane, Patrick A. C., 4852 Rothrist (CH)
(74) Representative: Tiefbrunner, Vera

(56) References cited:
- EP-A1- 2 264 108
- EP-A1- 2 264 109
- US-A- 5 814 312
- US-B1- 6 666 953

## Description

The present invention relates to a method for the control of odour, the use of surface-reacted calcium carbonate for the control of odour, as well as products for the control of odour.

Generally, odours are ubiquitous in the environment. While certain odours are perceived as pleasant, there are also others, malodours, causing an unpleasant sensation, and which, therefore, are continuously tried to be controlled by numerous ways.

One source of malodours, for example, is any kind of waste human and animal bodily excretions, liquids and secretions. However, there are also other sources of unpleasant odours which require to be controlled, such as those caused by food, e.g. from dairy products, meat and fish; or textiles; furniture; and walling.

As regards human or animal body liquids, there is a continuous need to control odour, and constant development to meet that need, e.g. in the field of personal hygiene articles such as sanitary napkins, panty liners, adult incontinence articles, infant diapers, paper towels, bath tissue and facial tissue, non wovens for medical purposes etc. Such articles are often used to absorb and retain bodily fluids and other exudates excreted by the human body.

For example, EP 0 510 619 A1 discloses a wide variety of materials which have proven to be effective in certain circumstances in reducing malodours in absorbent articles of personal hygiene. EP 0 959 846 A1 discloses such materials comprising polyacrylate superabsorbers and silica. EP 0 811 387 A1 discloses absorbent articles being provided with a zeolite and silica odour control system. EP 0 963 186 A1 discloses an odour control system comprising zeolites, silica and polyacrylic superabsorbers. EP 0 912 149 A1 discloses chelating agents for use in odour control in absorbent articles, particularly polyfunctionally substituted aromatic chelants.

US 5,814,312 A discloses a composition for reducing odors emanating from animal discharges consisting essentially of (a) water with a calcium carbonate content less than about 1000 ppm; (b) chlorine dioxide in an amount effective to reduce odors emanating from animal discharges; and (c) an acidulent in an amount sufficient to adjust the pH of the composition to a value greater than above 7.

Polymeric superabsorbers are able to absorb large volumes of liquid, but they are not very fast. Manufacturers of napkins and diapers are using superabsorber polymers for liquid absorbency, but potential for improvement is required in the speed of uptake to prevent initial leaking.

Furthermore, many of the presently used absorbents are rather specific and cannot be used for several malodourants simultaneously.

For solving this problem, combinations of absorbents, or absorbents and nose blocking agents, for example, were suggested. For example, EP 2 258 408 A1 discloses an absorbent article comprising an odour control system, wherein the odour control composition comprises two classes of odour control material, wherein a first class of odour control material such as silica gel, aldehydes or mesoporous zeolites, reduces odour by acting on malodours or a malodorous substance in the absorbent article and a second class of odour control material reduces odour by blocking the user's nose receptors due to the volatile nature of the materials selected, e.g. menthol.

Furthermore, e.g. as regards food related use, many known absorbents may not be used as they are hazardous to health, such that often odour control is simply managed by sealing the odour source, which may lead, however, to undesired accelerated degradation. Thus, it would be highly desirable to provide, e.g. wrapping paper or containers including non-hazardous material absorbing any malodourants, e.g. those produced by dairy products, meat or fish. The same applies to the control of odour, e.g. in the refrigerator.

There are further multiple uses for the control of odour such as to improve the room climate etc., wherein the most common approach to solve this problem presently is to exchange one odour by another such as by the use of room sprays etc. This, however, is not always desired, and a neutral odour control would be more desirable.

In this respect, it is to be noted that not simply any material being able to absorb, for example, liquids having an unpleasant odour are necessarily also suitable to control this odour, i.e. the volatile part of the odourant, in which case it is required to remove the composite of absorbent and absorbate to effect removal of the odour.

Ideally, agents for the control of odour can reduce the malodour according to different mechanisms, e.g. they can reduce the amount of malodorous molecules through absorption/adsorption mechanisms and/or can react with the malodorous molecules transforming them into low volatile/non-odorous ones and/or can suppress malodorous molecules by suppressing volatility and/or can prevent the malodour generation by inhibiting degradative processes caused by metabolic activity of microorganisms.

Thus, there are many fields of application for odour control, and a continuous need for new methods and agents therefor, in view of the fact that the known ones often are not suitable for the simultaneous control of several odours or are not applicable in certain fields, e.g. for safety or economic reasons.

It has now been found that calcium carbonate, which has been surface treated in a certain way has excellent ab/adsorption properties as regards many of the common malodours to be controlled, which is especially advantageous in view of the fact that calcium carbonate is a common and easily available material having no hazardous effects on health.

Thus, according to the present invention a new method for the control of odour is provided by contacting surface-reacted calcium carbonate with odourants, wherein the surface-reacted calcium carbonate is a reaction product of natural ground or precipitated calcium carbonate with carbon dioxide and one or more acids, wherein the carbon dioxide is formed in situ by the acid treatment and/or is supplied from an external source.

The calcium carbonate subjected to surface treatment may be natural ground calcium carbonate (GCC) or synthetic, i.e. precipitated calcium carbonate (PCC).

The natural ground calcium carbonate preferably is selected from calcium carbonate containing minerals selected from the group comprising marble, chalk, dolomite, limestone and mixtures thereof. Precipitated calcium carbonate is preferably selected from the group comprising precipitated calcium carbonates having aragonitic, vateritic or calcitic mineralogical crystal forms or mixtures thereof.

In a preferred embodiment, the natural or precipitated calcium carbonate is ground prior to the treatment with one or more acids and carbon dioxide. The grinding step can be carried out with any conventional grinding device such as a grinding mill known to the skilled person.

In a preferred process for the preparation the natural and synthetic calcium carbonate, either finely divided, such as by grinding, or not, is suspended in water. Preferably, the slurry has a content of natural or synthetic calcium carbonate within the range of 1 wt% to 80 wt%, more preferably 3 wt% to 60 wt%, and even more preferably 5 wt% to 40 wt%, based on the weight of the slurry.

In a next step, an acid, which, in the context of the present invention is a Brønsted acid, i.e. a H₃O⁺ ion donor, is added to the aqueous suspension containing the natural or synthetic calcium carbonate. Preferably, the acid has a pKₐ at 25°C of 2.5 or less. If the pKₐ at 25°C is 0 or less, the acid is preferably selected from sulphuric acid, hydrochloric acid, or mixtures thereof. If the pKₐ at 25°C is from 0 to 2.5, the acid is preferably selected from H₂SO₃, M⁺HSO₄⁻ (M⁺ is an alkali metal ion selected from the group comprising sodium and potassium, lithium or other Group I metals), H₃PO₄, oxalic acid or mixtures thereof.

The one or more acids can be added to the suspension as a concentrated solution or a more diluted solution. Preferably, the molar ratio of the acid to the natural or synthetic calcium carbonate is from 0.05 to 4, more preferably from 0.1 to 2.

As an alternative, it is also possible to add the acid to the water before the natural or synthetic calcium carbonate is suspended.

In a next step, the natural or synthetic calcium carbonate is treated with carbon dioxide. If a strong acid such as sulphuric acid or hydrochloric acid is used for the acid treatment of the natural or synthetic calcium carbonate, the carbon dioxide is automatically formed in a sufficient amount to achieve the required molar concentration. Alternatively or additionally, the carbon dioxide can be supplied from an external source.

Acid treatment and treatment with carbon dioxide can be carried out simultaneously which is the case when a strong acid is used. It is also possible to carry out acid treatment first, e.g. with a medium strong acid having a pKₐ in the range of 0 to 2.5, followed by treatment with carbon dioxide supplied from an external source.

Preferably, the concentration of gaseous carbon dioxide in the suspension is, in terms of volume, such that the ratio (volume of suspension): (volume of gaseous CO₂) is from 1:0.05 to 1:20, even more preferably 1:0.05 to 1:5.

In a preferred embodiment, the acid treatment step and/or the carbon dioxide treatment step are repeated at least once, more preferably several times.

Subsequent to the acid treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural or synthetic calcium carbonate as an aqueous suspension having a pH of greater than 6.0, preferably greater than 6.5, more preferably greater than 7.0, even more preferably greater than 7.5. If the aqueous suspension is allowed to reach equilibrium, the pH is greater than 7. A pH of greater than 6.0 can be adjusted without the addition of a base when stirring of the aqueous suspension is continued for a sufficient time period, preferably 1 hour to 10 hours, more preferably 1 to 5 hours.

Alternatively, prior to reaching equilibrium, which occurs at a pH greater than 7, the pH of the aqueous suspension may be increased to a value greater than 6 by adding a base subsequent to carbon dioxide treatment. Any conventional base such as sodium hydroxide or potassium hydroxide can be used.

Further details about the preparation of the surface-reacted natural calcium carbonate are disclosed in WO 00/39222 A1, WO 2004/083316 A1, WO 2005/121257 A2, WO 2009/074492 A1, EP 2 264 108 A1, EP 2 264 109 A1 and US 2004/0020410 A.

Surface-reacted calcium carbonate being useful in the present invention may also be prepared by contacting ground natural calcium carbonate with at least one water-soluble acid and with gaseous CO₂, wherein said acid(s) have a pKₐ of greater than 2.5 and less than or equal to 7, when measured at 20°C, associated with the ionisation of their first available hydrogen, and a corresponding anion formed on loss of this first available hydrogen capable of forming water-soluble calcium salts. Subsequently, at least one water-soluble salt, which in the case of a hydrogencontaining salt has a pKₐ of greater than 7, when measured at 20°C, associated with the ionisation of the first available hydrogen, and the salt anion of which is capable of forming water-insoluble calcium salts, is additionally provided.

In this respect, exemplary acids are acetic acid, formic acid, propanoic acid and mixtures thereof, exemplary cations of said water-soluble salt are selected from the group consisting of potassium, sodium, lithium and mixtures thereof, and exemplary anions of said water-soluble salt are selected from the group consisting of phosphate, dihydrogen phosphate, monohydrogen phosphate, oxalate, silicate, mixtures thereof and hydrates thereof.

Further details about the preparation of these surface-reacted natural calcium carbonates are disclosed in EP 2 264 108 A1 and EP 2 264 109 A1.

Similarly, surface-reacted precipitated calcium carbonate is obtained. As can be taken in detail from WO 2009/074492 A1, surface-reacted precipitated calcium carbonate is obtained by contacting precipitated calcium carbonate with H₃O⁺ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by H₃O⁺ ions, where said H₃O⁺ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said H₃O⁺ ions may be provided by the addition of an acid or an acid salt of said anion, or the addition of an acid or an acid salt which simultaneously serves to provide all or part of said excess solubilized calcium ions.

In a preferred embodiment of the preparation of the surface-reacted natural or synthetic calcium carbonate, the natural or synthetic calcium carbonate is reacted with the acid and/or the carbon dioxide in the presence of at least one compound selected from the group consisting of silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metal silicate. These components can be added to an aqueous suspension comprising the natural or synthetic calcium carbonate before adding the acid and/or carbon dioxide.

Alternatively, the silicate and/or silica and/or aluminium hydroxide and/or earth alkali aluminate and/or magnesium oxide component(s) can be added to the aqueous suspension of natural or synthetic calcium carbonate while the reaction of natural or synthetic calcium carbonate with an acid and carbon dioxide has already started. Further details about the preparation of the surface-reacted natural or synthetic calcium carbonate in the presence of at least one silicate and/or silica and/or aluminium hydroxide and/or earth alkali aluminate component(s) are disclosed in WO 2004/083316 A1.

The surface-reacted natural or synthetic calcium carbonate can be kept in suspension, optionally further stabilised by a dispersant. Conventional dispersants known to the skilled person can be used. A preferred dispersant is polyacrylic acid.

Alternatively, the aqueous suspension described above can be dried.

The surface-reacted natural or precipitated calcium carbonate to be used in the present invention preferably is provided in the dried powder form.

Furthermore, in a preferred embodiment, the surface-reacted natural or synthetic calcium carbonate has a specific surface area of from 1 m²/g to 200 m²/g, preferably 40 m²/g to 175 m²/g, more preferably 50 to 145 m²/g, especially preferably 60 m²/g to 90 m²/g, most preferably 70 m²/g to 80 m²/g, measured using nitrogen and the BET method according to ISO 9277.

It is preferred that the surface-reacted calcium carbonate has a volume median grain diameter *d*₅₀ of from 0.1 to 50 µm, preferably from 0.5 to 25 µm, more preferably 0.8 to 20 µm, particularly 1 to 10, e.g. 4 to 7 µm measured with a Malvern Mastersizer 2000 Laser Diffraction System. The method and the instrument are known to the skilled person and are commonly used to determine grain sizes of fillers and pigments.

Preferably, the surface-reacted calcium carbonate has an intra-particle intruded specific pore volume within the range of 0.150 to 1.300 cm³/g, and preferably of 0.178 to 1.244 cm³/g, calculated from mercury intrusion porosimetry measurement as described in the experimental section. The total pore volume seen in the cumulative intrusion data can be separated into two regions with the intrusion data from 214 µm down to about 1 - 4 µm showing the coarse packing of the sample between any agglomerate structures contributing strongly. Below these diameters lies the fine interparticle packing of the particles themselves. If they also have intraparticle pores, then this region appears bimodal. The sum of these three regions gives the total overall pore volume of the powder, but depends strongly on the original sample compaction/settling of the powder at the coarse pore end of the distribution.

By taking the first derivative of the cumulative intrusion curve the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired specific pore volume of the internal pores alone as the specific pore volume per unit mass. The same principle of subtraction, of course, applies for isolating any of the other pore size regions of interest.

The pore size of the surface-reacted calcium carbonate preferably is in a range of from 10 to 100 nm, more preferably in a range of between 20 and 80 nm, especially from 30 to 70 nm, e.g. 50 nm determined by mercury porosimetry measurement.

The surface-reacted calcium carbonate may be in the form of a powder and/or granules. It may also be in the form of suspensions or part of a gel, if appropriate. It is especially preferred that it is in the form of a powder and/or granules.

Granules may be prepared by common granulation processes selected from melt, dry or wet granulation processes as well as roller compaction.

An especially preferred method for the production of surface reacted calcium carbonate comprising granules is described in unpublished European patent application No. 14 170 578. According to this wet granulation method, the pores of the surface reacted calcium carbonate particles are first saturated with one or more granulation liquid, whereas one or more binders are added afterwards.

In this respect, the liquid may generally be any one commonly used in the field of granulation and is preferably water, wherein it does not act as an active ingredient having a specific effect in an organism and causing a specific reaction.

Liquid saturation may be achieved by adding the liquid to dry or not completely saturated surface-reacted calcium carbonate, or, if the surface-reacted calcium carbonate is provided as a suspension or filter cake, it may also be achieved by removing excess liquid. This may be done thermally or mechanically by techniques known to the person skilled in the art. The particles are defined to be liquid saturated, if the whole intraparticle pore volume of same is filled by the liquid.

Binders which may be used are those well-known in the art of granulation, such as carboxymethylcellulose or polyvinylpyrrolidone, and may also have disintegrating properties under certain conditions.

The one or more binder is added in dry form, or in the form of emulsions, dispersions, or solutions to the liquid saturated surface-reacted calcium carbonate in an amount of from 0.5 to 50 wt% based on the weight of surface-reacted calcium carbonate. It may be added to the agitation device simultaneously with or after the liquid saturated surface-reacted calcium carbonate, wherein it may be necessary to adjust the amount of binder, surface-reacted calcium carbonate and/or liquid saturated calcium carbonate, after the combination of the liquid saturated surface-reacted calcium carbonate and the one or more binder.

The mixture has the appropriate consistency as soon as the desired granule sizes, or granule size distribution, respectively, have been achieved, whereupon agitation may be continued.

The granulation equipment may be selected from the conventionally used ones for granulation purposes. Thus, the agitation device may be selected from the group comprising Eirich mixers, fluidized bed dryers/granulators/mixers, Lödige mixers, etc.

After the granulation process is completed, the liquid is removed by means of separating the liquid from the resulting granules.

The resulting granules may have a wide size range, wherein different size fractions may be separated by conventional means such as sieving.

Generally, the granules may have a volume median granule size of from 0.1 - 6 mm, preferably 0.2 - 5 mm and more preferably from 0.3 to 4 mm. Depending on the intended use of the granules size fractions of from 0.3 to 0.6 mm or 1 mm to 4 mm may be obtained, as well as grain sizes of from 0.6 to 1 mm or 1 to 2 mm determined by sieve fractioning.

The granules comprising surface reacted calcium carbonate may have a specific surface area of from 1 to 175 m²/g, preferably of from 2 to 145 m²/g, more preferably 10 to 100 m²/g, especially preferably of from 20 to 70 m²/g, most preferably of from 30 to 40 m²/g, measured using nitrogen and the BET method according to ISO 9277.

The granules obtained by the process according to the present invention have turned out to be more stable than those provided without binder or according to wet granulation without a previous liquid saturation of the surface-reacted calcium carbonate.

"Odour" according to the present invention generally is defined as one or more volatilized chemical compounds, generally at a very low concentration, that humans or other animals perceive by the sense of olfaction. Accordingly, an "odourant" is a chemical compound that has a smell or odour, i.e. is sufficiently volatile to be transported to the olfactory system in the upper part of the nose.

Preferred odours to be controlled according to the present invention are odours which cause an unpleasant sensation, i.e. malodours, but are not limited thereto.

Such odours may originate from odourants, which are preferably selected from the group comprising odourants contained in human and animal body liquids and secretion such as menses, blood, plasma, sanies; vaginal secretions, mucus, milk, urine; feces; vomit and perspiration; odourants originating from putrefaction such as of human or animal tissue; food such as dairy products, meat and fish; fruit such as durian fruit; textiles; furniture; car interiors; and walling.

Specifically, these odourants may be selected from the group comprising amines such as triethylamine, diethylamine, trimethylamine, diaminobutane, tetramethylenediamine, pentamethylenediamine, pyridine, indole, 3-methylindole; carboxylic acids such as propionic acid, butanoic acid, 3-methylbutanoic acid, 2-methylpropanoic acid, hexanoic acid; sulphur organic compounds such as thiols, e.g. methanethiol, phosphor organic compounds such as methylphosphine, dimethylphosphine; their derivatives and mixtures thereof.

Thus, it is an advantageous embodiment of the inventive method for the control of odour to use the surface reacted calcium carbonate in diapers, feminine hygiene products such as pads, panty liners, sanitary napkins, tampons; incontinence products; deodorant formulations; paper towels, bath tissue and facial tissue; nonwoven products such as wipes and medical products; packaging material, preferably plastic, paper or board packaging material, such as wrapping papers, packaging boards; mono and multilayer structures; permeable bags; ab/adsorption pads; paper products, preferably paper sheets, which may be filled and/or coated with surface-reacted calcium carbonate with or without adhesive layer; animal litter; construction and building material; preparations of compost and organic fertilizers.

Accordingly, it is a further aspect of the present invention to provide a product for the control of odour, which contains surface reacted calcium carbonate, and is preferably selected from the group comprising diapers, feminine hygiene products such as pads, panty liners, sanitary napkins and tampons; incontinence products; deodorant formulations; nonwoven products such as wipes and medical products; plastic packaging material; mono and multilayer structures; permeable bags; ab/adsorption pads; animal litter; construction and building material; preparations of compost and organic fertilizers.

In this respect, it is generally possible to include the surface reacted calcium carbonate in any well-known products in the form of a separate layer in multilayer systems or as additive in existing, e.g. liquid absorbing layers, as a filler, e.g. in plastics, e.g. wrapping paper, or as a coating, in the form of bags, or any other form allowing the contact of the odourant and/or its volatile phase with the surface reacted calcium carbonate.

It might also be advantageous to print surface reacted calcium carbonate, e.g. by means of inkjet printing, flexography, or gravure printing, onto material such as paper.

The following figures, examples and tests will illustrate the present invention, but are not intended to limit the invention in any way.

### Description of the figures:

- Figure 1: illustrates the results of ab/adsorption trials of triethylamine using several known ab/adsorbents and surface reacted calcium carbonate powder according to the invention.
- Figure 2: illustrates the results of ab/adsorption trials of diethylamine and triethylamine using several known ab/adsorbents and surface reacted calcium carbonate granules according to the invention.
- Figure 3: illustrates the results of ab/adsorption trials of butanoic acid, 3-methylbutanoic acid and hexanoic acid using several known ab/adsorbents and surface reacted calcium carbonate granules according to the invention.
- Figure 4: illustrates the results of ab/adsorption trials of butanoic acid using several known ab/adsorbents and surface reacted calcium carbonate powders and granules according to the invention.
- Figure 5: illustrates the results of ab/adsorption trials of butanoic acid using several known ab/adsorbents and surface reacted calcium carbonate powders in dependence of the specific surface areas.
- Figure 6: illustrates the results of smell intensity evaluation trials of urine in diapers with and without surface reacted calcium carbonate.
- Figure 7: illustrates the results of hedonic evaluation trials of urine in diapers with and without surface reacted calcium carbonate.

### EXAMPLES

### 1. Measurement methods

The following measurement methods were used to evaluate the parameters given in the examples and claims.

### BET specific surface area (SSA) of a material

The BET specific surface area was measured via the BET process according to ISO 9277 using nitrogen, following conditioning of the sample by heating at 250°C for a period of 30 minutes. Prior to such measurements, the sample was filtered, rinsed and dried at 110°C in an oven for at least 12 hours.

### Particle size distribution (volume % particles with a diameter < X), d₅₀ value (volume median grain diameter) and d₉₈ value of a particulate material:

Volume median grain diameter *d*₅₀ was evaluated using a Malvern Mastersizer 2000 Laser Diffraction System. The *d*₅₀ or *d*₉₈ value, measured using a Malvern Mastersizer 2000 Laser Diffraction System, indicates a diameter value such that 50 % or 98 % by volume, respectively, of the particles have a diameter of less than this value. The raw data obtained by the measurement are analysed using the Mie theory, with a particle refractive index of 1.57 and an absorption index of 0.005.

The weight median grain diameter is determined by the sedimentation method, which is an analysis of sedimentation behaviour in a gravimetric field. The measurement is made with a Sedigraph™ 5100, Micromeritics Instrument Corporation. The method and the instrument are known to the skilled person and are commonly used to determine grain size of fillers and pigments. The measurement is carried out in an aqueous solution of 0.1 wt% Na₄P₂O₇. The samples were dispersed using a high speed stirrer and supersonicated.

The processes and instruments are known to the skilled person and are commonly used to determine grain size of fillers and pigments.

### Porosity / Pore volume

The porosity or pore volume is measured using a Micromeritics Autopore IV 9500 mercury porosimeter having a maximum applied pressure of mercury 414 MPa (60 000 psi), equivalent to a Laplace throat diameter of 0.004 µm (∼ nm). The equilibration time used at each pressure step is 20 seconds. The sample material is sealed in a 5 ml chamber powder penetrometer for analysis. The data are corrected for mercury compression, penetrometer expansion and sample material compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 35(5), 1996, p1753-1764.).

### 2. Material and equipment

### 2.1. Equipment

- Sorption tube (Sigma Aldrich, stainless steel, 1/4 in x 3 1/2 in)
- Thermal desorption tube with Tenax^{®} TA (Sigma Aldrich, stainless steel, 1/4 in x 3 1/2 in)
- Pocket Pump 210-1000 series (of SKC Inc., Eighty Four, PA, USA)
- TD-GC-MS (thermal desorption-gas chromotograph-mass spectrometer):
   *TD TurboMatrix Perkin Elmer*
      Temperature Mode: Tube 300 °C, Valve 195 °C, Transfer 200 °C, Trap Low -20°C, Trap High 300°C
      Timing: Desorb 10 min, Purge 1.0 min, Trap Hold 5.0 min
      Pneumatic Settings: Column 95 kPa, Outlet Split 50 ml/min, Inlet Split 40 ml/min, Desorb 30mL/min
   *GC Method AutoSystem XL Perkin Elmer*
      Column: Optima 5 Accent 1.0 µm, 60m * 0.32 mm, Macherey-Nagel
      Temperature of oven: 110°C for 15 min (amines)
      Temperature of oven: 130°C for 10 min (acids)
   *MS Turbo Mass Perkin Elmer*
      Solvent Delay 0.0 min
      Full Scan 25 to 350 m/z (mass/charge) (EI+)

### 2.2. Material

### Absorbents

- Millicarb OG (Omya AG; natural ground calcium carbonate; *d*₅₀ = 3 µm)
- Kaolin clay (Sigma-Aldrich; CAS 1332-58-7)
- Sea sand (silica) (CAS 60676-86-0)
- Vermiculite (BET specific surface area: 4.3 m²/g)
- Diatomite (BET specific surface area: 5.6 m²/g)
- Activated Carbon (BET specific surface area: 1400 m²/g)

### - Surface-reacted calcium carbonate (SRCC) powder 1 (d₅₀ = 4.3 µm, d₉₈ = 8.6 µm, SSA = 51.6 m²g⁻¹)

SRCC 1 was obtained by preparing 8 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 20 wt%, based on the total weight of the aqueous suspension, is obtained. Whilst mixing the slurry, 1.22 kg of an aqueous solution containing 30 wt% phosphoric acid and 0.4% aluminium sulphate hexadecahydrate was added to said suspension over a period of 10 minutes at a temperature of 70°C. After the addition of the solution, the slurry was stirred for an additional 5 minutes, before removing it from the vessel and drying.

### - Surface-reacted calcium carbonate (SRCC) powder 2 (d₅₀ = 3.7 µm, d₉₈ = 8.1µm, SSA = 72.7 m²g⁻¹)

SRCC 2 was obtained by preparing 8 litres of an aqueous suspension of wet ground calcium carbonate, containing polyacrylate dispersant added in the grinding process, in a mixing vessel by adjusting the solids content of a ground marble calcium carbonate from Omya Hustadmarmor AS having a mass based particle size distribution with 90 w/w% of the particles finer than 2 µm, as determined by sedimentation, such that a solids content of 20 wt%, based on the total weight of the aqueous suspension, is obtained.

Whilst mixing the slurry, 1.2 kg of an aqueous solution containing 30 wt% phosphoric acid was added to said suspension over a period of 10 minutes at a temperature of 70°C. Simultaneous to the start of the phosphoric acid solution addition, 0.92 kg of an aqueous solution containing 10 wt% sodium silicate was added to said suspension over a period of 14 minutes. After the addition of the two solutions, the slurry was stirred for an additional 5 minutes, before removing it from the vessel and drying.

### - Surface-reacted calcium carbonate (SRCC) powder 3 (d₅₀ = 3.5 µm, d₉₈ = 7.6 µm, SSA = 92.3 m²g⁻¹)

SRCC 3 was obtained by preparing 8 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 20 wt%, based on the total weight of the aqueous suspension, is obtained.

Whilst mixing the slurry, 1.2 kg of an aqueous solution containing 30 wt% phosphoric acid was added to said suspension over a period of 10 minutes at a temperature of 70°C. Two minutes after the start of the phosphoric acid solution addition, 0.37 kg of an aqueous solution containing 25 wt% citric acid was added to said suspension over a period of 0.5 minutes. After the addition of the two solutions, the slurry was stirred for an additional 5 minutes, before removing it from the vessel and drying.

### - Surface-reacted calcium carbonate (SRCC) powder 4 (d₅₀ = 5.5 µm, d₉₈ = 10.6 µm, SSA = 141.5 m²g⁻¹)

SRCC 4 was obtained by preparing 10 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 16 wt%, based on the total weight of the aqueous suspension, is obtained.

Whilst mixing the slurry, 3 kg of an aqueous solution containing 30 wt% phosphoric acid was added to said suspension over a period of 10 minutes at a temperature of 70°C. Two minutes after the start of the phosphoric acid solution addition, 0.36 kg of an aqueous solution containing 25 wt% citric acid was added to said suspension over a period of 0.5 minutes. After the addition of the two solutions, the slurry was stirred for an additional 5 minutes, before removing it from the vessel and drying.

### - Surface-reacted calcium carbonate (SRCC) powder 5 (d₅₀ = 5.1 µm, d₉₈ =9.8 µm, SSA = 51.4 m²g⁻¹)

SRCC 5 was obtained by preparing 10 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 15 wt%, based on the total weight of the aqueous suspension, is obtained.

Whilst mixing the slurry, 1.7 kg of an aqueous solution containing 30 wt% phosphoric acid was added to said suspension over a period of 10 minutes at a temperature of 70°C. After the addition of the solution, the slurry was stirred for an additional 5 minutes, before removing it from the vessel and drying.

### - Surface-reacted calcium carbonate (SRCC) powder 6 (d₅₀ = 5.0 µm, d₉₈ = 9.6 µm, SSA = 62.2 m²g⁻¹)

SRCC 6 was obtained by preparing 10 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 15 wt%, based on the total weight of the aqueous suspension, is obtained.

Whilst mixing the slurry, 1.7 kg of an aqueous solution containing 30 wt% phosphoric acid was added to said suspension over a period of 10 minutes at a temperature of 70°C. Four minutes after starting the addition of phosphoric acid solution, 41 g of an aqueous solution containing trisodium citrate was added in a separate stream to the slurry over a period of 0.5 minutes. After the addition of the solution, the slurry was stirred for an additional 5 minutes, before removing it from the vessel and drying.

### - Surface-reacted calcium carbonate (SRCC) powder 7 (d₅₀ = 5.1 µm, d₉₈ = 9.8 µm, SSA = 77.1 m²g⁻¹)

SRCC 7 was obtained by preparing 10 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 15 wt%, based on the total weight of the aqueous suspension, is obtained.

Whilst mixing the slurry, 1.7 kg of an aqueous solution containing 30 wt% phosphoric acid was added to said suspension over a period of 10 minutes at a temperature of 70°C. Simultaneous to the start of the phosphoric acid solution addition, 0.88 kg of an aqueous solution containing 7.5 wt% sodium silicate was added to said suspension over a period of 10 minutes. After the addition of the two solutions, the slurry was stirred for an additional 5 minutes, before removing it from the vessel and drying.

### - Surface-reacted calcium carbonate (SRCC) powder 8 (d₅₀ = 4.4 µm, d₉₈ = 8.6 µm, SSA = 39.9 m²g⁻¹)

SRCC 8 was obtained by preparing 10 litres of an aqueous suspension of ground calcium carbonate in a mixing vessel by adjusting the solids content of a wet ground marble calcium carbonate, containing polyacrylate dispersant added in the grinding process, from Omya Hustadmarmor AS having a mass based particle size distribution with 90 w/w% of the particles finer than 2 µm, as determined by sedimentation, such that a solids content of 15 wt%, based on the total weight of the aqueous suspension, is obtained.

Whilst mixing the slurry, 0.83 kg of an aqueous solution containing 30 wt% phosphoric acid was added to said suspension over a period of 10 minutes at a temperature of 70°C. After the addition of the solution, the slurry was stirred for an additional 5 minutes, before removing it from the vessel and drying.

### - Surface-reacted calcium carbonate granules 1

As a starting material for granulation the following surface-reacted calcium carbonate powder SRCC 9 (*d*₅₀ = 6.6 µm, *d*₉₈ = 13.7 µm, SSA = 59.9 m²g⁻¹) was prepared:
350 litres of an aqueous suspension of ground calcium carbonate in a mixing vessel was prepared by adjusting the solids content of a wet ground limestone calcium carbonate, containing dispersant added in the grinding process from Omya SAS, Orgon, having a mass based median particle size of 1.3 µm, as determined by sedimentation, such that a solids content of 10 wt%, based on the total weight of the aqueous suspension, is obtained.

Whilst mixing the slurry at a speed of 6.2 m/s, 11.2 kg phosphoric acid was added in form of an aqueous solution containing 30 wt% phosphoric acid to said suspension over a period of 20 minutes at a temperature of 70°C. After the addition of the acid, the slurry was stirred for additional 5 minutes, before removing it from the vessel and drying using a jet-dryer.

The intra-particle intruded specific pore volume of this surface-reacted calcium carbonate is 0.939 cm³/g (for the pore diameter range of 0.004 to 0.51 µm).

400 g of this surface-reacted calcium carbonate was added to a Lödige mixer (Model L5, 5 liter, Gebr. Lödige Maschinenbau GmbH, Paderborn, Germany). Subsequently, 400 g of a solution containing 3 wt% sodium carboxymethylcellulose (Sigma Aldrich (average molar mass 90000 g/mol; CAS No. 9004-32-4) in water was added using a spray bottle while mixing the powder with both the blending element (speed varied between 500 rpm and the maximum speed (999 rpm), mainly between 700 - 999 rpm) and the cutter until the material started to look a little clumpy. After this point, the sample turned into a paste. This was again rectified via the addition of 100 g of dry surface-reacted calcium carbonate. The sample was mixed a few more minutes until individual granules were formed. Subsequently, the sample was removed and dried at 90°C for 12 hours.

### - Surface-reacted calcium carbonate granules 2 and 3

530 g of SRCC 9 powder were saturated with water providing a solids content of 61 wt% and added to the Lödige mixer. Subsequently, 26 g sodium carboxymethylcellulose (Sigma Aldrich (average molar mass 90000 g/mol; CAS No. 9004-32-4) was added, dry, and the combination was mixed for several minutes to ensure proper blending. Subsequently, using a spray bottle, tap water was added over time, while mixing the powder with both the blending element (speed varied between 500 rpm and the maximum speed (999 rpm), mainly between 700 - 999 rpm) and the cutter until the material started to look a little clumpy. At this point, a little more water was then added and the sample turned to a paste. This was again rectified via the addition of 100 g dry surface-reacted calcium carbonate. The sample was mixed a few more minutes until individual granules were formed. The final solids of this sample was 65 wt%. Subsequently, the sample was removed and dried at 90°C for 12 hours.

The dried sample was sieved on a Retsch sieve into separate size fractions, namely < 0.3 mm, between 0.3 and 0.6 mm, between 0.6 and 1 mm, and between 1 and 2 mm.

For further trials the following fractions were used, where x is the particle size of the granules:
Surface reacted calcium carbonate granules 2: 0.6 mm < *x* < 1 mm
Surface reacted calcium carbonate granules 3: 0.3 mm < *x* < 0.6 mm

### Odourants

- Diethylamine (Sigma Aldrich, CAS 109-89-7)
- Triethylamine (Sigma Aldrich, CAS 121-44-8)
- Butanoic acid (Sigma Aldrich, CAS 107-92-6)
- 3-Methylbutanoic acid (Sigma Aldrich, CAS 503-74-25)
- Hexanoic acid (Sigma Aldrich, CAS 142-62-1)

### 3. Ab/adsorption Trials

### 3.1. Surface reacted calcium carbonate powders

A stock solution of 1500 mg/l triethylamine in water was prepared.

For carrying out the ab/adsorption trials the ab/adsorption tube was filled with:

| | |
|---|---|
| Example 1: | No ab/adsorbent |
| Example 2: | 0.4 g of a mixture of 1 g sea sand and 0.5 g Millicarb OG |
| Examples 3 - 6: | 0.4 g of a mixture of 1 g sea sand and 0.5 g SRCC powders 1 to 4, respectively |

In front of the sorption tube, a vial filled with 10 µl of the prepared triethylamine based stock solution was installed, behind the tube a thermal desorption tube with a Tenax TA.

During 5 min. air was sucked from the odourant filled vial through both of the tubes by means of a pocket pump (SKC) at a rate of 80 ml/min at room temperature (23 °C). Subsequently, the odourant content in the Tenax TA tube was analysed by means of TD-GC-MS.

The area under the detected peak proportionally corresponds to the odourant concentration. Therefore, odourant ab/adsorption by different materials can be compared by means of the peak area.

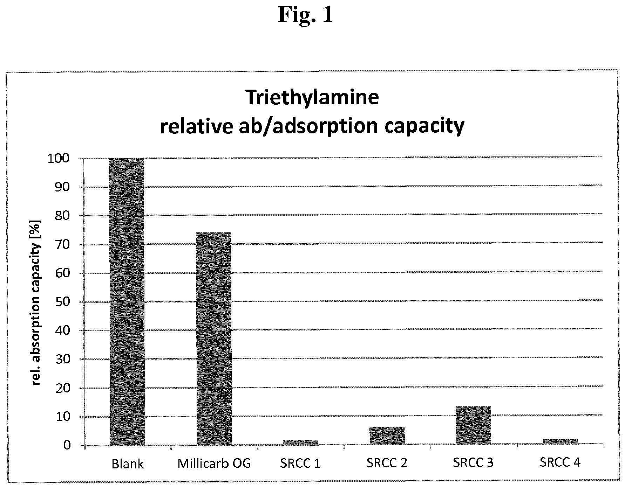

The trials were repeated several times. The average values obtained are summarized in figure 1 reflecting the resulting relative ab/adsorption capacity of the respective samples, wherein 100 % refers to the maximum value determined for the blank sample (Example 1).

As can be clearly seen from these results, there is only a very poor ab/adsorption of triethylamine by conventional natural ground calcium carbonate (Millicarb OG). The result, however, looks quite different using powders of surface-reacted calcium carbonate, showing much higher levels of sorption.

Based on these findings further experiments were carried out using further odourants and surface reacted calcium carbonate granules.

### 3.2. Surface reacted calcium carbonate granules

The following stock solutions were prepared having the respective given concentrations in water:

| | |
|---|---|
| Diethylamine: | 3000 mg/l |
| Triethylamine: | 1500 mg/l |
| Butanoic acid: | 1000 mg/l |
| 3-Methylbutanoic acid: | 1000 mg/l |
| Hexanoic acid: | 1600 mg/l |

For carrying out the ab/adsorption trials the sorption tube was filled with:

| | |
|---|---|
| Example 7: | No ab/adsorbent |
| Example 8: | 0.4 g of a mixture of 1 g sea sand and 0.5 g Millicarb OG |
| Example 9: | 0.4 g of a mixture of 1 g sea sand and 0.5 g kaolin clay |
| Example 10: | 0.4 g surface reacted calcium carbonate granules 1 |

In front of the absorption tube, a vial filled with 10 µl of the respective stock solution was installed, behind the tube a thermal desorption tube with a Tenax TA.

During 5 min. air was sucked from the odourant filled vial through both of the tubes by means of a pocket pump (SKC) at a rate of 80 ml/min at room temperature (23 °C). Subsequently, the odourant content in the Tenax TA tube was analyzed by means of TD-GC-MS.

The area under the detected peak proportionally corresponds to the odourant concentration. Therefore, odourant ab/adsorption by different materials can be compared by means of the peak area.

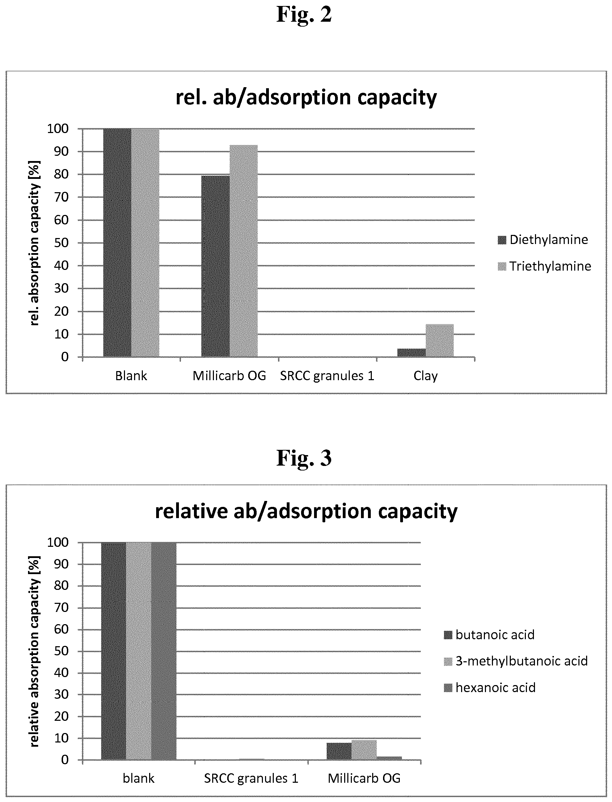

The trials were repeated several times. The average values obtained are summarized in figures 2 and 3 reflecting the resulting relative absorption capacity of the respective samples, wherein 100 % refers to the maximum value determined for the blank sample (Example 7).

As can be clearly observed from these results, there is only a very poor ab/adsorption of diethylamine and triethylamine by conventional natural ground calcium carbonate (Millicarb OG). Contrary to this, these odourants are essentially completely ab/adsorbed by the surface reacted calcium carbonate granules.

As regards butanoic acid, 3-methylbutanoic acid and hexanoic acid, which are quite well adsorbed by kaolin clay, these results can even be improved by using surface reacted calcium carbonate granules.

Furthermore, contrary to, e.g. natural ground calcium carbonate or kaolin clay, surface reacted calcium carbonate granules 1 are able to ab/adsorb odourants of different chemical classes equally well.

Apart from these quantitative results, an olfactory test was carried out, wherein it turned out that the sample of surface reacted calcium carbonate granules having butanoic acid, 3-methylbutanoic acid, and hexanoic acid absorbed into or adsorbed on it did not have any smell, whereas the sample kaolin clay had an unpleasant smell.

This finding led to further olfactory tests described further below.

### 3.3. Further butanoic acid ab/adsorption trials

As butanoic acid is one of the most unpleasant odours in the food sector, further evaluations were made regarding this odourant.

A stock solution of butanoic acid was prepared having a concentration in water of 5 wt%.

For carrying out the sorption trials the sorption tube was filled with:

| | |
|---|---|
| Example 11: | No ab/adsorbent |
| Example 12: | Silica gel |
| Example 13: | Vermiculite |
| Example 14: | Diatomite |
| Example 15: | Activated Carbon |
| Example 16: | Kaolin clay |
| Example 17: | Millicarb OG |
| Example 18 - 21: | SRCC powders 5 to 8, respectively |
| Examples 22 + 23: | Surface reacted calcium carbonate (SRCC) granules 2 and 3, respectively |

0.2000 g of glass wadding and 0.8000 g of the respective ab/adsorbent (except for the granules, which were used alone) were weighed into a beaker. The mixture was cut with scissors until a homogeneous mixture was obtained. 60 mg of the absorbent/glass wadding mixture, or 48 mg of SRCC granules 2 or 3, respectively, were filled into the sorption tube. In front of the sorption tube, a vial filled with 10 µl of the butanoic acid stock solution was installed, behind the tube a thermal desorption tube with a Tenax TA. After the solution was filled with the stock solution, it was heated in a water bath at 40 °C. Subsequently, during 5 min. air was sucked from the odourant filled vial through both of the tubes by means of a pocket pump (SKC) at a rate of 80 ml/min at 40 °C. Subsequently, the odourant content in the Tenax TA tube was analysed by means of TD-GC-MS.

The area under the detected peak proportionally corresponds to the odourant concentration. Therefore, odourant ab/adsorption by different materials can be compared by means of the peak area.

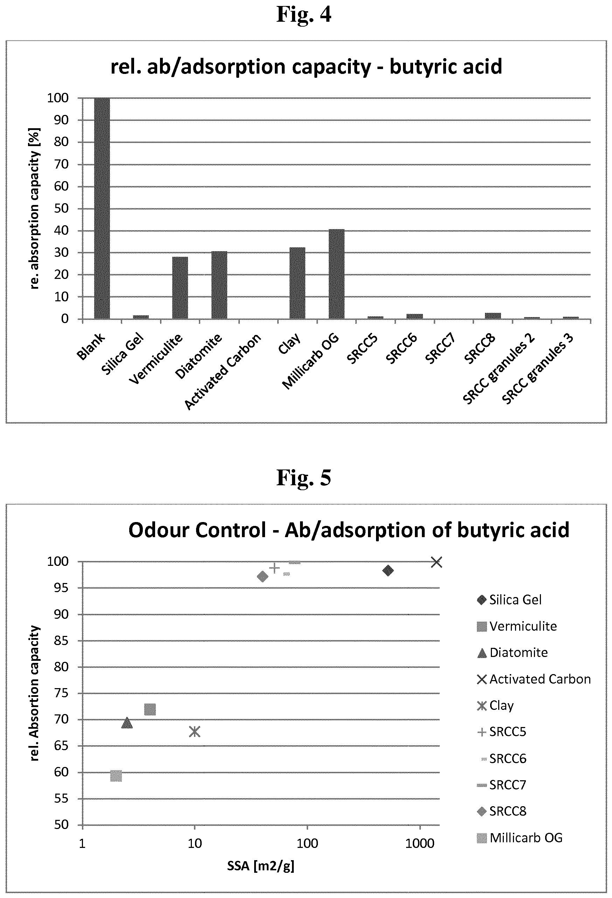

The trials were repeated several times. The average values obtained are summarized in figure 4 reflecting the resulting relative ab/adsorption capacity of the respective samples, wherein 100 % refers to the maximum value determined for the blank sample (Example 11).

As can be seen from figure 4 the sorption capacity of any one of the SRCC powders and granules is significantly better than conventional natural ground calcium carbonate as well as vermiculite, diatomite and kaolin clay.

As regards silica gel and activated carbon, the surface reacted calcium carbonate powders provide at least comparable values, wherein it has to be noted that silica gel as well as activated carbon have a considerably higher specific surface area, such that the sorption capacity per surface area is significantly higher than the one of silica gel and activated carbon (cf. figure 5).

### 4. Olfactory Trials

In the following trials comparative human sensory measurements of the smell of urine in an incontinence product were carried out. For this purpose products with and without the surface reacted calcium carbonate according to the invention were compared as regards the parameters of smell intensity (strong smell / no smell) and hedonic evaluation (pleasant / unpleasant).

### 4.1. Measurement methods

Smell may be described by several smell parameters. The determination of these parameters is described in different guidelines, the following of which were used:
- Intensity (VDI 3882; Olfactometry; determination of odour intensity; technical Rule, Publication date: 1992-10; Beuth Verlag)
- Hedonic smell impression (VDI 3882 and ISO 16000-28)

The smell measurements were performed by 12 test persons and one supervisor. The testing team was trained and selected according to DIN EN 13725:2003 [1].

### 4.1.1. Smell Intensity

The evaluation of the intensity is carried out by means of a category scale from "not perceptible" (0) to "extremely strong" (6) according to VDI 3882.

For evaluating the smell intensity the test person assigned his smell impression to the following terms given in table 1:

**Table 1**

| **Smell** | **Intensity category scale** |
|---|---|
| Extremely strong | 6 |
| Very strong | 5 |
| Strong | 4 |
| Considerable | 3 |
| Weak | 2 |
| Very weak | 1 |
| Not perceptible | 0 |

In this respect, level 1 is assigned if the odour detection threshold is exceeded, which means that the test person was sure that a smell was noticed, even if it could not be clearly assigned to a certain smell quality.

Subsequently, the arithmetic average of the respective individual evaluations of the group of test persons was calculated.

### 4.1.2. Hedonic smell impression

The hedonic evaluation describes whether a smell impression was a pleasant or unpleasant sensation. For evaluating the hedonic smell impression, the following smell scale was used:

| | | | | | | | | |
|---|---|---|---|---|---|---|---|---|
| -4 | -3 | -2 | -1 | 0 | +1 | +2 | +3 | +4 |
| Extremely unpleasant | | Neither pleasant nor unpleasant | | | | | Extremely pleasant | |

Subsequently, the arithmetic average of the respective individual evaluations of the group of test persons was calculated.

### 4.2. Experimental procedure

### 4.2.1 Material

The following material was used:
- 2 cloth diapers (100 % cotton; Alana; available from dm-drogerie markt GmbH + Co. KG)
- Nalophan PET odour sample bags (available from Odournet GmbH)
- PureSniff device (available from Odournet GmbH)
- 20 g of surface-reacted calcium carbonate (SRCC) powder 10 (*d*₅₀ = 6.1 µm, *d*₉₈ = 14.2 µm, SSA = 144.0 m²g⁻¹)

SRCC 10 was obtained by preparing 350 litres of an aqueous suspension of ground calcium carbonate in a mixing vessel by adjusting the solids content of a wet ground marble calcium carbonate, containing dispersant added in the grinding process, from Omya Avenza SPA having a mass based particle size distribution with 90 w/w% of the particles finer than 2 µm, as determined by sedimentation, such that a solids content of 16 wt%, based on the total weight of the aqueous suspension, is obtained. Whilst mixing the slurry, 104 kg of an aqueous solution containing 30 wt% phosphoric acid was added to said suspension over a period of 10 minutes at a temperature of 70°C. Two minutes after the start of the phosphoric acid solution addition, 12.5 kg of an aqueous solution containing 25 wt% citric acid was added to said suspension over a period of 0.5 minutes.

After the addition of the two solutions, the slurry was stirred for an additional 5 minutes, before removing it from the vessel and drying.

### - A mixture of urine (including first morning urine) of three probands

### 4.2.2 Sample Preparation

A diaper was folded once, and 20 g of surface reacted calcium carbonate was poured onto it. The diaper was folded again to cover the surface reacted calcium carbonate. A second diaper was folded likewise without containing surface reacted calcium carbonate.

For simulating the urination, the diapers were each placed into a Nalophan bag having a volume of 601, and 10 ml of urine was emptied onto each of the diapers.

Further simulated urinations were performed after 2 hours (10 ml) and 4 hours (5 ml). The bags were filled up with air and stored in a climate chamber at 36 °C. Smell samples were taken after 1 min, 1 h, 2 h, 3 h, 4 h, 6 h and 8 h.

### 4.2.3 Sample evaluation

After taking a sample, the sample was transferred into a PureSniff device generating a constant and highly repeatable odour sample volume flow at the outlet (nose mask) of the device. In this way, each olfactory test person receives an identical sample with a standardized volume flow over a constant presentation time, ensuring repeatable conditions for the odour evaluation. At any time, each sample was evaluated by each test person twice in randomized order. Thus, in total each sample was evaluated (N =) 24 times at any time of sample taking.

### 4.3. Results

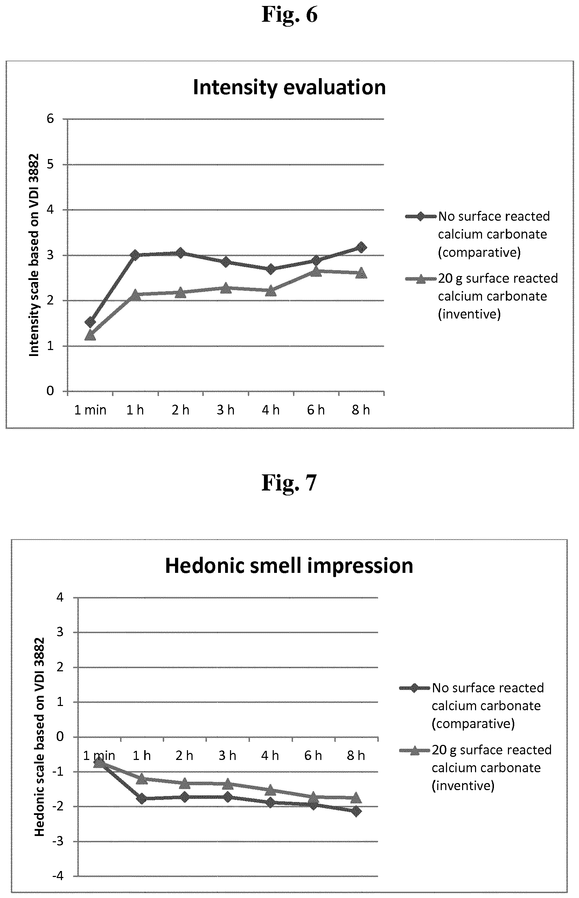

The total average values of the intensity evaluation are summarized in table 2 and illustrated in figure 6:

**Table 2**

| *N***=*24*** | **No surface reacted calcium carbonate (comparative)** | **20 g surface reacted calcium carbonate (inventive)** |
|---|---|---|
| 1 min | 1.52 | 1.25 |
| 1 h | 3.00 | 2.13 |
| 2 h | 3.05 | 2.18 |
| 3 h | 2.85 | 2.28 |
| 4 h | 2.69 | 2.22 |
| 6 h | 2.88 | 2.65 |
| 8 h | 3.17 | 2.61 |

The total average values of the hedonic evaluation are summarized in table 3 and illustrated in figure 7:

**Table 3**

| ***N=*24** | **No surface reacted calcium carbonate (comparative)** | **20 g surface reacted calcium carbonate (inventive)** |
|---|---|---|
| 1 min | - 0.73 | - 0.73 |
| 1 h | - 1.78 | - 1.20 |
| 2 h | - 1.73 | - 1.33 |
| 3 h | - 1.73 | - 1.35 |
| 4 h | - 1.89 | - 1.53 |
| 6 h | - 1.95 | - 1.73 |
| 8 h | - 2.14 | - 1.75 |

These results very clearly show that the use of surface reacted calcium carbonate in diapers containing urine, not only provide a reduction of the smell intensity, but also an improvement of the hedonic impression.

## Claims

1. Method for the control of odour by contacting surface-reacted calcium carbonate with odourants, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source.

2. Method according to claim 1,
**characterized in that** the natural ground calcium carbonate is selected from calcium carbonate containing minerals selected from the group comprising marble, chalk, dolomite, limestone and mixtures thereof; and that the precipitated calcium carbonate is selected from the group comprising precipitated calcium carbonates having aragonitic, vateritic or calcitic mineralogical crystal forms or mixtures thereof.

3. Method according to any one of claims 1 or 2,
**characterized in that** the surface-reacted calcium carbonate is in the form of a powder and/or granules, suspension and/or gels, preferably in the form of a powder and/or granules.

4. The process according to any one of the preceding claims,
**characterised in that** the surface-reacted calcium carbonate has a specific surface area of from 1 m²/g to 200 m²/g, preferably 40 m²/g to 175 m²/g, more preferably 50 to 145 m²/g, especially preferably 60 m²/g to 90 m²/g, most preferably 70 m²/g to 80 m²/g, measured using nitrogen and the BET method according to ISO 9277.

5. The process according to any one of the preceding claims,
**characterised in that** the surface-reacted calcium carbonate particles have a volume median grain diameter *d*₅₀ of from 0.1 to 50 µm, preferably from 0.5 to 25 µm, more preferably 0.8 to 20 µm, particularly 1 to 10, e.g. 4 to 7 µm.

6. The process according to any one of the preceding claims,
**characterized in that** the surface-reacted calcium carbonate has an intra-particle intruded specific pore volume within the range of 0.150 to 1.300 cm³/g, and preferably of 0.178 to 1.244 cm³/g, calculated from a mercury intrusion porosimetry measurement.

7. The process according to any one of the preceding claims,
**characterised in that** the surface reacted calcium carbonate is in the form of granules having a volume median granule size of from 0.1 - 6 mm, preferably from 0.2 - 5 mm, more preferably from 0.3 to 4 mm, especially preferably from 0.3 to 0.6 mm or 1 mm to 4 mm, most preferably from 0.6 to 1 mm or 1 to 2 mm determined by sieve fractioning.

8. The method according to any one of the preceding claims,
**characterized in that** the surface reacted calcium carbonate is in the form of granules having a specific surface area of from 1 to 175 m²/g, preferably of from 2 to 145 m²/g, more preferably 10 to 100 m²/g, especially preferably of from 20 to 70 m²/g, most preferably of from 30 to 40 m²/g, measured using nitrogen and the BET method according to ISO 9277.

9. Method according to any one of the preceding claims,
**characterized in that** the odourants are selected from the group comprising odourants contained in human and animal body liquids and secretion such as menses, blood, plasma, sanies, vaginal secretions, mucus, milk, urine, feces, vomit; and perspiration; odourants originating from putrefaction such as of human or animal tissue; food such as dairy products, meat and fish; fruit; textiles; furniture; car interiors; and walling.

10. Method according to any one of the preceding claims,
**characterized in that** the odourants are selected from the group comprising amines such as triethylamine, diethylamine, trimethylamine, diaminobutane, tetramethylenediamine, pentamethylenediamine, pyridine, indole, 3-methylindole; carboxylic acids such as propionic acid, butanoic acid, 3-methylbutanoic acid, 2-methylpropanoic acid, hexanoic acid; sulphur organic compounds such as thiols, e.g. methanethiol, phosphor organic compounds such as methylphosphine, dimethylphosphine; their derivatives and mixtures thereof.

11. Method according to any one of the preceding claims,
**characterized in that** the surface reacted calcium carbonate is used in diapers, feminine hygiene products such as pads, panty liners, sanitary napkins and tampons; incontinence products; deodorant formulations; paper towels, bath tissue and facial tissue; nonwoven products such as wipes and medical products; packaging material, preferably plastic, paper or board packaging material, such as wrapping papers, packaging boards; mono and multilayer structures; permeable bags; adsorption pads; paper products, preferably paper sheets, filled and/or coated with surface-reacted calcium carbonate with or without adhesive layer; animal litter; construction and building material; preparations of compost and organic fertilizers.

12. Use of the surface-reacted calcium carbonate as defined in any one of claims 1 to 8 for the control of odour.

13. Product for the control of odour,
**characterized in that** it contains surface reacted calcium carbonate as defined in any one of claims 1 to 8 for the control of odour, wherein the surface-reacted calcium carbonate is a reaction product of natural ground or precipitated calcium carbonate with carbon dioxide and one or more acids in an aqueous medium, wherein the carbon dioxide is formed in situ by the acid treatment and/or is supplied from an external source; and
wherein the product is selected from diapers, feminine hygiene products such as pads, panty liners, sanitary napkins and tampons; incontinence products; deodorant formulations; nonwoven products such as wipes and medical products; plastic packaging material; mono and multilayer structures; permeable bags; adsorption pads; animal litter; construction and building material; preparations of compost and organic fertilizers.

## Patentansprüche

1. Verfahren zur Geruchskontrolle durch Inkontaktbringen eines Calciumcarbonats, das an der Oberfläche eine Reaktion eingegangen ist, mit Geruchsstoffen, wobei das Calciumcarbonat, das an der Oberfläche eine Reaktion eingegangen ist, ein Reaktionsprodukt von natürlichem, vermahlenem oder gefälltem Calciumcarbonat mit Kohlenstoffdioxid und einer oder mehreren Säuren in einem wässrigen Medium ist, wobei das Kohlenstoffdioxid in situ durch die Säurebehandlung gebildet wird und/oder aus einer externen Quelle zugeführt wird.

2. Verfahren nach Anspruch 1,
**dadurch gekennzeichnet, dass** das natürliche, vermahlene Calciumcarbonat ausgewählt ist aus Calciumcarbonat umfassenden Mineralien, ausgewählt aus der Gruppe umfassend Marmor, Kreide, Dolomit, Kalkstein sowie Gemische davon; und dass das gefällte Calciumcarbonat ausgewählt ist aus der Gruppe umfassend gefällte Calciumcarbonate mit aragonitischen, vateritischen oder calcitischen mineralogischen Kristallformen oder Gemischen davon.

3. Verfahren nach einem der Ansprüche 1 oder 2,
**dadurch gekennzeichnet, dass** das Calciumcarbonat, das an der Oberfläche eine Reaktion eingegangen ist, in der Form eines Pulvers und/oder von Granulaten, Suspension und/oder Gelen ist, bevorzugt in der Form eines Pulvers und/oder von Granulaten.

4. Verfahren nach einem der vorhergehenden Ansprüche,
**dadurch gekennzeichnet, dass** das Calciumcarbonat, das an der Oberfläche eine Reaktion eingegangen ist, eine spezifische Oberfläche von 1 m²/g bis 200 m²/g aufweist, bevorzugt 40 m²/g bis 175 m²/g, bevorzugter 50 m²/g bis 145 m²/g, besonders bevorzugt 60 m²/g bis 90 m²/g, am meisten bevorzugt 70 m²/g bis 80 m²/g, gemessen durch Verwenden von Stickstoff und dem BET-Verfahren gemäss ISO 9277.

5. Verfahren nach einem der vorhergehenden Ansprüche,
**dadurch gekennzeichnet, dass** die Partikel des Calciumcarbonats, das an der Oberfläche eine Reaktion eingegangen ist, einen volumengemittelten Korndurchmesser *d*₅₀ von 0,1 bis 50 µm aufweisen, bevorzugt von 0,5 bis 25 µm, bevorzugter 0,8 bis 20 µm, insbesondere 1 bis 10, z.B. 4 bis 7 µm.

6. Verfahren nach einem der vorhergehenden Ansprüche,
**dadurch gekennzeichnet, dass** das Calciumcarbonat, das an der Oberfläche eine Reaktion eingegangen ist, ein spezifisches Intrapartikelporeneindringvolumen im Bereich von 0,150 bis 1,300 cm³/g aufweist, und bevorzugt von 0,178 bis 1,244 cm³/g, berechnet aus einer Quecksilberporosimetriemessung.

7. Verfahren nach einem der vorhergehenden Ansprüche,
**dadurch gekennzeichnet, dass** das Calciumcarbonat, das an der Oberfläche eine Reaktion eingegangen ist, in der Form von Granulaten mit einer volumengemittelten Granulatgrösse von 0,1 bis 6 mm ist, bevorzugt von 0,2 bis 5 mm, bevorzugter von 0,3 bis 4 mm, besonders bevorzugt von 0,3 bis 0,6 mm oder 1 mm bis 4 mm, am meisten bevorzugt von 0,6 bis 1 mm oder 1 bis 2 mm, bestimmt durch Siebfraktionierung.

8. Verfahren nach einem der vorhergehenden Ansprüche,
**dadurch gekennzeichnet, dass** das Calciumcarbonat, das an der Oberfläche eine Reaktion eingegangen ist, in der Form von Granulaten mit einer spezifischen Oberfläche von 1 bis 175 m²/g ist, bevorzugt von 2 bis 145 m²/g, bevorzugter 10 bis 100 m²/g, besonders bevorzugt von 20 bis 70 m²/g, am meisten bevorzugt von 30 bis 40 m²/g, gemessen durch Verwenden von Stickstoff und dem BET-Verfahren gemäss ISO 9277.

9. Verfahren nach einem der vorhergehenden Ansprüche,
**dadurch gekennzeichnet, dass** die Geruchsstoffe ausgewählt sind aus der Gruppe umfassend Geruchsstoffe, die enthalten sind in menschlichen und tierischen Körperflüssigkeiten und Ausscheidungen, wie Menstruation, Blut, Plasma, eitrigen Absonderungen, Vaginalsekreten, Schleim, Milch, Urin, Fäkalien, Erbrochenem; und Schweissabsonderung; Geruchsstoffe, die entstammen aus Fäulnis, wie von menschlichem oder tierischem Gewebe; in Nahrungsmitteln, wie Molkereiprodukten, Fleisch und Fisch; Obst; Textilien; Möbeln; Fahrzeuginnenräumen; sowie Gemäuern.

10. Verfahren nach einem der vorhergehenden Ansprüche,
**dadurch gekennzeichnet, dass** die Geruchsstoffe ausgewählt sind aus der Gruppe umfassend Amine, wie Triethylamin, Diethylamin, Trimethylamin, Diaminobutan, Tetramethylendiamin, Pentamethylendiamin, Pyridin, Indol, 3-Methylindol; Carbonsäuren, wie Propionsäure, Buttersäure, 3-Methylbuttersäure, 2-Methylpropionsäure, Capronsäure; organische Schwefelverbindungen, wie Thiole, z.B. Methanthiol, organische Phosphorverbindungen, wie Methylphosphin, Dimethylphosphin; deren Derivate, sowie Gemische davon.

11. Verfahren nach einem der vorhergehenden Ansprüche,
**dadurch gekennzeichnet, dass** das Calciumcarbonat, das an der Oberfläche eine Reaktion eingegangen ist, verwendet wird in Windeln, Damenhygieneprodukten, wie Pads, Slipeinlagen, Damenbinden und Tampons; Inkontinenzprodukten; Deodorantformulierungen; Papiertüchern, Toilettenpapier und Kosmetiktüchern; Vliesprodukten, wie Wischtücher und Medizinprodukte; Verpackungsmaterial, bevorzugt Kunststoff-, Papier- oder Kartonverpackungsmaterial, wie Packpapier, Verpackungskartons; Mono- und Mehrschichtstrukturen; permeablen Beuteln; Adsorptionspads; Papierprodukten, bevorzugt Papierbögen, gefüllt und/oder gestrichen mit Calciumcarbonat, das an der Oberfläche eine Reaktion eingegangen ist, mit oder ohne Klebschicht; Tierstreu; Baustoff und Baumaterial; Kompostzubereitungen sowie organischen Düngemitteln.

12. Verwendung des Calciumcarbonats, das an der Oberfläche eine Reaktion eingegangen ist, wie in einem der Ansprüche 1 bis 8 definiert, zur Geruchskontrolle.

13. Produkt für die Geruchskontrolle,
**dadurch gekennzeichnet, dass** es Calciumcarbonat, das an der Oberfläche eine Reaktion eingegangen ist, wie in einem der Ansprüche 1 bis 8 definiert, zur Geruchskontrolle enthält, wobei das Calciumcarbonat, das an der Oberfläche eine Reaktion eingegangen ist, ein Reaktionsprodukt von natürlichem, vermahlenem oder gefälltem Calciumcarbonat mit Kohlenstoffdioxid und einer oder mehreren Säuren in einem wässrigen Medium ist, wobei das Kohlenstoffdioxid in situ durch die Säurebehandlung gebildet wird und/oder aus einer externen Quelle zugeführt wird; und
wobei das Produkt ausgewählt ist aus Windeln, Damenhygieneprodukten, wie Pads, Slipeinlagen, Damenbinden und Tampons; Inkontinenzprodukten; Deodorantformulierungen; Vliesprodukten, wie Wischtücher und Medizinprodukte; Kunststoffverpackungsmaterial; Mono- und Mehrschichtstrukturen; permeablen Beuteln; Adsorptionspads; Tierstreu; Baustoff und Baumaterial; Kompostzubereitungen sowie organischen Düngemitteln.

## Revendications

1. Procédé de contrôle d'odeurs par la mise en contact de carbonate de calcium ayant réagi en surface avec des odorisants, dans lequel le carbonate de calcium ayant réagi en surface est un produit réactionnel de carbonate de calcium naturel broyé ou précipité avec du dioxyde de carbone et d'un ou plusieurs acides dans un milieu aqueux, dans lequel le dioxyde de carbone est formé in situ par le traitement acide et/ou est fourni à partir d'une source externe.

2. Procédé selon la revendication 1, **caractérisé en ce que** le carbonate de calcium naturel broyé est choisi parmi des minéraux contenant du carbonate de calcium choisis dans le groupe comprenant le marbre, la craie, la dolomite, le calcaire et des mélanges de ceux-ci ; et **en ce que** le carbonate de calcium précipité est choisi dans le groupe comprenant les carbonates de calcium précipités ayant des formes cristallines minéralogiques aragonitiques, vatéritiques ou calcitiques ou des mélanges de ceux-ci.

3. Procédé selon l'une quelconque des revendications 1 à 2, **caractérisé en ce que** le carbonate de calcium ayant réagi en surface est sous la forme d'une poudre et/ou de granulés, d'une suspension et/ou de gels, de préférence sous la forme d'une poudre et/ou de granulés.

4. Procédé selon l'une quelconque des revendications précédentes, **caractérisé en ce que** le carbonate de calcium ayant réagi en surface a une surface spécifique de 1 m²/g à 200 m²/g, de préférence de 40 m²/g à 175 m²/g, plus préférablement de 50 m²/g à 145 m²/g, en particulier de 60 m²/g à 90 m²/g, de manière préférée entre toutes de 70 m²/g à 80 m²/g, mesurée en utilisant de l'azote et le procédé BET selon la norme ISO 9277.

5. Procédé selon l'une quelconque des revendications précédentes, **caractérisé en ce que** le carbonate de calcium ayant réagi en surface a un diamètre de grain médian en volume *d₅₀* de 0.1 à 50 µm, de préférence de 0.5 à 25 µm, plus préférablement de 0.8 à 20 µm, en particulier de 1 à 10, par exemple de 4 à 7 µm.

6. Procédé selon l'une quelconque des revendications précédentes, **caractérisé en ce que** le carbonate de calcium ayant réagi en surface a un volume de pore spécifique immiscé intraparticulaire compris dans la plage allant de 0.150 à 1.300 cm³/g, et de préférence de 0.178 à 1.244 cm³/g, calculé à partir d'une mesure de porosimétrie par intrusion de mercure.

7. Procédé selon l'une quelconque des revendications précédentes, **caractérisé en ce que** le carbonate de calcium ayant réagi en surface est sous la forme de granulés ayant une taille de granulé médiane en volume de 0.1 à 6 mm, de préférence de 0.2 à 5 mm, plus préférablement de 0.3 à 4 mm, en particulier de 0.3 à 0.6 mm ou de 1 mm à 4 mm, de manière préférée entre toutes de 0.6 à 1 mm ou de 1 à 2 mm, déterminée par fractionnement sur tamis.

8. Procédé selon l'une quelconque des revendications précédentes, **caractérisé en ce que** le carbonate de calcium ayant réagi en surface est sous la forme de granulés ayant une surface spécifique de 1 à 175 m²/g, de préférence de 2 à 145 m²/g, plus préférablement de 10 à 100 m²/g, en particulier de 20 à 70 m²/g, de manière préférée entre toutes de 30 à 40 m²/g, mesurée en utilisant de l'azote et le procédé BET selon la norme ISO 9277.

9. Procédé selon l'une quelconque des revendications précédentes, **caractérisé en ce que** les odorants sont choisis dans le groupe comprenant les odorants contenus dans les liquides et les sécrétions corporels d'êtres humains et d'animaux tels que les menstruations, le sang, le plasma, les sécrétions vaginales, le mucus, le lait, l'urine, les selles, le vomi ; et la transpiration ; les odorants provenant de la putréfaction tels que d'un tissu humain ou animal ; les aliments tels que les produits laitiers, la viande et le poisson ; les fruits ; les textiles ; les meubles ; les intérieurs de voitures ; et les murs.

10. Procédé selon l'une quelconque des revendications précédentes, **caractérisé en ce que** les odorants sont choisis dans le groupe comprenant les amines telles que la triéthylamine, la diéthylamine, la triméthylamine, le diaminobutane, la tétraméthylènediamine, la pentaméthylènediamine, la pyridine, l'indole, le 3-méthylindole ; les acides carboxyliques tels que l'acide propionique, l'acide butanoïque, l'acide 3-méthylbutanoïque, l'acide 2-méthylpropanoïque, l'acide hexanoïque ; les composés organiques soufrés tels que les thiols, par exemple le méthanethiol, les composés organiques phosphorés tels que la méthylphosphine, la diméthylphosphine ; des dérivés et des mélanges de ceux-ci.

11. Procédé selon l'une quelconque des revendications précédentes, **caractérisé en ce que** le carbonate de calcium ayant réagi en surface est utilisé dans des couches, des produits d'hygiène féminine tels que des tampons, des protège-slips, des serviettes et des tampons hygiéniques; des produits d'incontinence ; des formulations déodorantes ; des serviettes en papier, du papier hygiénique et des mouchoirs en papier ; des produits non tissés tels que les lingettes et les produits médicaux ; un matériau d'emballage, de préférence un matériau en plastique, papier ou carton, tel que des papiers d'emballage, des cartons d'emballage ; des structures mono et multicouches ; des sacs perméables ; des tampons d'adsorption ; des produits en papier, de préférence des feuilles de papier, chargés et/ou revêtus de carbonate de calcium ayant réagi en surface avec ou sans couche adhésive ; une litière pour animaux ; des matériaux de construction et pour le bâtiment ; des préparations de compost et d'engrais organiques.

12. Utilisation du carbonate de calcium ayant réagi en surface tel que défini selon l'une quelconque des revendications 1 à 8 pour la régulation d'odeurs.

13. Produit de régulation d'odeurs, **caractérisé en ce qu'**il contient du carbonate de calcium ayant réagi en surface tel que défini selon l'une quelconque des revendications 1 à 8 pour le contrôle d'odeurs, dans lequel le carbonate de calcium ayant réagi en surface est un produit réactionnel de carbonate de calcium naturel broyé ou précipité avec du dioxyde de carbone et d'un ou plusieurs acides dans un milieu aqueux, dans lequel le dioxyde de carbone est formé in situ par le traitement acide et/ou est fourni à partir d'une source externe ; et
dans lequel le produit est choisi parmi les couches, les produits d'hygiène féminine tels que les tampons, les protège-slips, les serviettes et les tampons hygiéniques; les produits d'incontinence ; les formulations déodorantes ; les produits non tissés tels que les lingettes et les produits médicaux ; un matériau d'emballage en plastique ; les structures mono et multicouches ; les sacs perméables ; les tampons d'adsorption ; une litière pour animaux ; les matériaux de construction et pour le bâtiment ; les préparations de compost et d'engrais organiques.
